# EUROPÄISCHE PATENTSCHRIFT

(11) **EP 1 017 998 B1**
(45) Veröffentlichungstag und Bekanntmachung des Hinweises auf die Patenterteilung: **15.04.2009**
(21) Anmeldenummer: 98955321.9
(22) Anmeldetag: 14.09.1998
(51) Int. Cl.: G01N 27/00

(54) **MIKROSTRUKTURIERTER BIOSENSOR, VERWENDUNG DES BIOSENSORS UND VERFAHREN ZUR IMMOBILISIERUNG VON BIOKATALYSATOREN**
MICRO-STRUCTURED BIO-SENSOR, USE OF SAID BIO-SENSOR AND METHOD FOR IMMOBILISING BIO-CATALYSTS
BIOCAPTEUR MICROSTRUCTURE, UTILISATION DE CE BIOCAPTEUR ET PROCEDE D'IMMOBILISATION DE BIOCATALYSEURS

(30) Priorität: 26.09.1997 DE 19742690
(43) Veröffentlichungstag der Anmeldung: 12.07.2000
(73) Patentinhaber: SPHERE Medical Limited, Cambridge Cambridgeshire CB2 5GG (GB)
(72) Erfinder: STANZEL, Manfred, D-91056 Erlangen (DE); GUMBRECHT, Walter, D-91074 Herzogenaurach (DE)
(74) Vertreter: Gillard, Richard Edward
(86) Internationale Anmeldenummer: PCT/DE1998/002719
(87) Internationale Veröffentlichungsnummer: WO 1999/017107

(56) Entgegenhaltungen:
- EP-A- 0 216 467
- EP-A- 0 415 124
- EP-A- 0 588 153
- EP-A- 0 636 879
- EP-A- 0 872 729
- GB-A- 2 230 865
- P VADGAMA, P W CRUMP: "Biosensors: Recent Trends" ANALYST, Bd. 117, November 1992, Seiten 1657-1670, XP002096648 in der Anmeldung erwähnt
- F SCHELLER, F SCHUBERT, D PFEIFFER: "Enzym- und Zellsensoren - Anwendungen, Trends und Perspektiven" SPEKTRUM DER WISSENSCHAFT, Bd. 9, September 1992, Seiten 99-103, XP002096649 in der Anmeldung erwähnt

## Beschreibung

Die Erfindung betrifft einen mikrostrukturierten, planaren Biosensor, insbesondere einen Biosensor mit einem Enzym als Biokatalysator. Weiterhin betrifft die Erfindung die Verwendung des Biosensors sowie ein Verfahren zur Immobilisierung von Biokatalysatoren.

Es gibt bereits Analysengeräte mit Erkennungsschichten aus Enzymmembranen oder Enzymschichten (die beiden Begriffe werden hier synonym gebraucht), mittels denen die durch einen enzymatischen Umsatz, beispielsweise von Sauerstoff, verursachte Änderung der Wasserstoffperoxid- oder Sauerstoffkonzentration elektrochemisch oder amperometrisch bestimmbar ist (F. Scheller et. al., "Enzym- und Zellsensoren - Anwendungen, Trends und Perspektiven", Spektrum der Wissenschaft, September 1992, Seiten 99 bis 103). Die Funktionalität, beispielsweise das Sättigungsverhalten und die Empfindlichkeit, eines solchen Biosensors ist in erster Linie von der Qualität der verwendeten Enzymmembran abhängig. Bei der Herstellung der Membran müssen mehrere Kriterien erfüllt werden (P. Vadgama und P. W. Crump, "Biosensors: Recent Trends", Analyst, November 1992, Vol. 117, Seiten 1657 bis 1670). Es muß eine möglichst große Menge aktiver Enzyme so immobilisiert oder gebunden werden, daß sie weitgehend funktionsfähig und aktiv bleibt und gleichzeitig in der Membran verhaftet ist. Um diese Bindung der Enzyme in der Membran möglichst schonend zu erreichen, sind eine Reihe von Techniken erarbeitet worden. Es kommen folgende Methoden zum Einsatz: Adsorption, ionische Bindung, Absorption, Einschluß in Mikrokapseln oder in Membranen und kovalente Bindung an Trägersubstanzen.

Die Herstellung der Enzymmembranen erfolgt zum einen durch physikalische Bindung der Enzyme in polymeren "Gelen", wie Poly(hydroxyethylmethacrylat), in denen die Enzyme wie in Netzen gehalten werden. Nachteilig daran ist, daß dabei immer auch Wechselwirkungen zwischen den Gelmolekülen und den Enzymproteinen entstehen, die die Flexibilität der dreidimensionalen Struktur und damit auch die Aktivität der Enzyme nachteilig beeinflussen. Andererseits werden auch Techniken angewandt, mittels denen die Enzymproteine direkt an polymere Träger über kovalente chemische Bindungen angehängt werden. Dabei wird durch Crosslinking, d.h. Vernetzung, zwischen den Aminosäureseitenketten des Enzyms und den aktiven Gruppen des Trägers natürlich erst recht die Dynamik der Tertiärstruktur des Proteins beeinflußt. Diese Struktur ist jedoch eine wichtige Aktivitätsvoraussetzung des Enzyms, weshalb eine solche Einschränkung der Strukturflexibilität mit einer teilweisen Inaktivierung des Enzyms einhergeht.

Aus der GB 2230865 ist eine struktur einer Glucosesensorelektrode bekannt. In diser Elektrode ist eine Schicht aus Polyurethan oder Polyhydroxymethacrylate und Glucoseoxidase ist mit einer zweiter Schicht, die aus einer Mischung von hydrophobern Polyurethan und hydroplile Polyurethan oder Polyhydroxymethacrylate besteht bedekt.

Aus EP 216 467 ist eine Anordnung eines sensors bekannt, bei dem ein Enzymsubstrate mit schichten unterschiedliche Dürchlässigheit ungeben ist.

Aus EP 415124 ist eine Enzymelektrode behannt, bei der eine innere membrane mit Enzyme und Coenzyme von einer aüßere membrane ungeben ist.

Aus der EP 0 588 153 B1 ist eine Struktur eines planaren, mikrostrukturierten Gassensorchips mit einer ersten, inneren und einer zweiten, äußeren Begrenzungsstruktur bekannt. Diese Struktur ermöglicht die Ausbildung einer Topf-Deckel-Struktur für eine Meßlösung (Elektrolytlösung) dadurch, daß innerhalb der ersten, inneren Begrenzungsstruktur die Elektrolytlösung (Topf) eingebracht wird, und innerhalb der zweiten, äußeren Begrenzungsstruktur ein hydrophobes und abschirmendes Material (Deckel) so eingebracht wird, daß es die Elektrolytlösung, zusammen mit der ersten, inneren Begrenzungsstruktur, vollständig bedeckt. Mit diesem planaren Sensorchip werden elektrochemisch Gase, wie Sauerstoff und Kohlendioxid, bestimmt.

Für biosensorische Bestimmungen mit einem Sensorchip dieser Art gab es bislang keine Topf-Deckel-Struktur, weil als Erkennungsschicht ("Meßlösung") nur die Enzymmembran, die aus einer einzigen homogenen Schicht gebildet ist, zur Verfügung stand. Die beiden Begrenzungsstrukturen des Chips konnten also bislang für biosensorische Bestimmungen nicht ausgenutzt werden, weil zur Ausbildung der Topf-Deckel-Struktur zwei Schichten anstelle einer einzigen homogenen Schicht vorliegen müssen.

Aufgabe der vorliegenden Erfindung ist es daher, einen Biosensor mit verbesserter Funktionalität zur Verfügung zu stellen, dessen Erkennungsschicht aus zwei Schichten besteht und in der die Dynamik der Tertiärstruktur der Enzyme unverändert bleibt. Weiterhin ist es Aufgabe der Erfindung, Anwendungen für einen solchen Biosensor zu finden. Schließlich ist es Aufgabe der Erfindung, ein Verfahren zur Verfügung zu stellen, mit dem eine Topf-Deckel-Struktur auf einem Sensorchip mit einer inneren und einer äußeren Begrenzungsstruktur herstellbar ist.

Gegenstand der Erfindung ist daher ein planarer Biosensor nach Anspruch 1, bei dens de biomolekulare Umsatz, der gemessen werden soll, elektrochemisch bestimmbar ist, wobei der Sensor zumindest zwei Begrenzungsstrukturen auf einem planaren Substrat umfaßt:
- eine erste, innere Begrenzungsstruktur, die mit einer Enzymmembran angefüllt ist, und
- eine zweite, äußere Begrenzungsstruktur, die mit einer polymeren Abdeckschicht, die die erste Begrenzungsstruktur mir der Enzymmembran vollständig bedeckt, angefüllt ist.

Außerdem ist Gegenstand der Erfindung die Verwendung des Biosensors in der Medizin, nämlich bei der minimal-invasiven Bestimmung von Leitmetaboliten, wie Glucose und Lactat, im Schließlich ist Gegenstand der Erfindung ein Verfahren nach Anspruch 7 zur Immobilisierung von Biokatalysatoren bei dem in einem ersten Schritt der Biokatalysator in eitner flüssigen Lösung auf ein Substrat in eine erste, innere Begrenzungsstruktur eingefüllt wird, in einem zweiten Schritt dort getrocknet wird und Schließlich in einem dritten Schritt mit einer polymerisierbaren Abdeckschicht überzogen wird.
Vorteilhafte Ausgestaltungen der Erfindung ergeben sich aus den Unteransprüchen und der Beschreibung.

Bevorzugt wird als Biokatalysator ein Enzym, besonders bevorzugt eines, das als Cosubstrat (Coenzym) Sauerstoff braucht, wie Glucoseoxidase oder Lactatoxidase, eingesetzt.

Bevorzugt enthält die Enzymmembran eine Pufferlösung, die Triethylenglykol und einen Phosphatpuffer umfaßt. Ein Beispiel dafür ist eine Lösung aus 5000 U Glucoseoxidase, 120 µl eine= Phosphatpufferlösung (Konzentration 100 mmol/l, pH 7) und 30 µl Triethylenglykol.

Außerdem ist es vorteilhaft, wenn der Enzymmembran eine planarisierende Hilfssubstanz, wie Polyvinylpyrrolidon (PVP), zugesetzt wird.

Vor dem Aufbringen der Abdeckschicht muß die Lösung mit der Enzymmembran oder Enzymschicht so getrocknet sein, daß sie gelartig oder kristallin vorliegt. Die fertig vernetzte Abdeckschicht ist wasserdurchlässig, so daß die Enzymschicht im Betriebszustand des Biosensors wieder feucht sein kann, damit die Enzyme aktiv sind.

Bevorzugt wird als Abdeckschicht eine UV-vernetzende Deckschicht eingesetzt. Ein Bespiel dafür ist ein photostrukturierbares Hydroxyethylmethacrylat-Harz (HEMA-Harz), bestehend aus einer Lösung von 2,374 g HEMA, 0,025 g Dimethoxyphenylacetophenon (DMAP) und 0,075 g Tetraethylenglykolmethacrylat (TEGM) in 60 µl Triethylenglykol. Es können auch andere polymerisierbare Substanzen eingesetzt werden, solange sie unter Bedingungen härten oder auspolymerisieren, die der Biokatalysator unbeschadet verträgt. Die fertige, d.h. auspolymerisierte Deckschicht muß so beschaffen sein, daß sie für alle an der Reaktion beteiligten Stoffe, außer dem Biokatalysator, durchlässig ist. Beispielsweise muß sie, wenn Glucose bestimmt werden soll, für Wasser, Sauerstoff und Glucose durchlässig sein, nicht aber für das Enzym Glucoseoxidase.

Bevorzugt liegt der Biokatalysator in der Membran möglichst trocken, d.h. entweder kristallin oder gelartig, vor. Als kristallin wird hier eine Enzymmembran bezeichnet, die trocken und planar ist.

Die Begrenzungsstrukturen sind Polymerstrukturen, die beispielsweise aus einem Kunststoff wie Polyimid gebildet sein können. Die Begrenzungsstrukturen können unterschiedlich hoch oder gleich hoch sein.

Die Erfindung ermöglicht eine wesentliche Verbesserung der Funktionalität von Biosensoren, weil sich das Biomolekül ohne störende Wechselwirkung im "Topf" bewegen kann und wegen des "Deckels" in der Meßlösung gehalten wird. Dies zeigt sich ganz besonders im verbesserten Sättigungsverhalten (Messungen bis zu einer Glucosekonzentration von ca. 35 mmol/l sind möglich) und in der gesteigerten Sensorempfindlichkeit (Stromdichten von > 100 nA·l/mmol·mm² bei Konzentrationen von < 20 mmol/l Glucose) des erfindungsgemäßen Biosensors.

Die erreichte Empfindlichkeit und das Sättigungsverhalten lassen einen Einsatz der Erfindung sowohl im medizinischphysiologischen Rahmen zu, der zwischen 3 und 20 mol/l (Konzentration an zu messender Substanz) liegt, als auch in der Biotechnologie, bei der - je nach Anwendungsfall - die Anforderungen bis zu einem Bereich von 100 mmol/l und in Extremfällen auch darüber reichen.

## Patentansprüche

1. Planarer Biosensor, bei dem der zu messende biomolekulare Umsatz elektrochemisch bestimmbar ist, wobei der Sensor zumindest zwei Begrenzungsstrukturen auf einem planaren Substrat umfaßt:
- eine erste, innere Begrenzungsstruktur, die eine Enzymschicht enthält, und
- eine zweite, äußere Begrenzungsstruktur, die mit einer polymeren wasserdurchlässigen Abdeckschicht, die die erste Begrenzungsstruktur mit der Enzymschicht vollständig bedeckt, angefüllt ist,
und wobei das Enzym in der Enzymschicht trocken vorliegt.

2. Biosensor nach Anspruch 1, bei dem das Enzym als Cosubstrat Sauerstoff benötigt.

3. Biosensor nach Anspruch 1 oder 2, bei dem in der Enzymschicht eine Pufferlösung enthalten ist, die Triethylenglykol und einen Phosphatpuffer umfaßt.

4. Biosensor nach einem der Ansprüche 1 bis 3, bei dem die Abdeckschicht ein UV-vernetztes Polymer ist.

5. Biosensor nach Anspruch 4, bei dem die Abdeckschicht ein vernetztes Hydroxyethylmethacrylat-Harz ist.

6. Verwendung des Biosensors nach einem oder mehreren der Ansprüche 1 bis 5 zur minimal-invasiven Bestimmung von Glucose oder Lactat im Blut.

7. Verfahren zur Immobilisierung von Biokatalysatoren, bei dem ein Biokatalysator in einem ersten Schritt in flüssiger Lösung in eine erste, innere Begrenzungsstruktur auf einem Substrat eingefüllt wird, in einem zweiten Schritt getrocknet wird, wobei er trocken und planar vorliegt, und schließlich in einem dritten Schritt mit einer polymerisierbaren wasserdurchlässigen Abdeckschicht überzogen wird.

## Claims

1. Planar biosensor, in which the biomolecular conversion to be measured can be determined electrochemically, wherein the biosensor comprises at least two limiting structures on a planar substrate:
- a first, inner limiting structure containing the first enzyme layer, and
- a second, outer limiting structure which is filled with a water-permeable polymer cover layer which completely covers the first limiting structure containing the enzyme layer,
and wherein the enzyme in the enzyme layer is dry.

2. Biosensor according to claim 1, wherein the enzyme requires oxygen as a cosubstrate.

3. Biosensor according to either claim 1 or claim 2, wherein a buffer solution containing triethylene glycol and a phosphate buffer is contained in the enzyme layer.

4. Biosensor according to any one of claims 1 to 3, wherein the cover layer is a UV cross-linked polymer.

5. Biosensor according to claim 4, wherein the cover layer is a cross-linked hydroxyethyl methacrylate resin.

6. Use of the biosensor according to any one of claims 1 to 5 to determine glucose or lactate levels in the blood in a minimally invasive manner.

7. Method of immobilising biocatalysts, wherein in a first step a biocatalyst in a liquid solution is used to fill a first, inner limiting structure on a substrate, and is dried in a second step so as to be present in a dry and planar manner, and is finally covered by a polymerisable water-permeable cover layer in a third step.

## Revendications

1. Biocapteur planaire, où le taux de métabolisme biomoléculaire à mesurer est déterminable par voie électrochimique, le capteur comprenant au moins deux structures limitatives sur un substrat planaire :
- une première structure limitative interne qui contient une couche d'enzyme et
- une deuxième structure limitative externe qui est remplie d'une couche de revêtement polymérique perméable à l'eau, qui recouvre entièrement la première structure limitative comportant une couche d'enzyme,
- et où l'enzyme dans la couche d'enzyme se présente sous forme sèche.

2. Biocapteur selon la revendication 1, où l'enzyme a besoin d'oxygène comme co-substrat.

3. Biocapteur selon les revendications 1 ou 2, où la couche d'enzyme contient une solution tampon qui comprend du triéthylèneglycol et un tampon phosphate.

4. Biocapteur selon l'une des revendications 1 à 3, où la couche de revêtement est un polymère réticulé aux UV.

5. Biocapteur selon la revendication 4, où la couche de revêtement est une résine réticulée d'hydroxyéthyl méthacrylate.

6. Utilisation du biocapteur selon une ou plusieurs des revendications 1 à 5 afin de déterminer de façon minimalement invasive le taux de glucose et de lactate dans le sang.

7. Procédé d'immobilisation de biocatalyseurs, où dans une première étape un biocatalyseur est introduit en solution liquide dans une première structure limitative interne sur un substrat, dans une deuxième étape il est séché, étant ainsi disponible sous forme séchée et planaire, puis finalement dans une troisième étape il est recouvert d'une couche de revêtement perméable à l'eau et polymérisable.
